Europäisches Patentamt

(19) European Patent Office

Office européen des brevets

(11) Publication number: **0 030 444**
**B1**

(12) **EUROPEAN PATENT SPECIFICATION**

(45) Date of publication of patent specification: **30.01.85**

(51) Int. Cl.⁴: **A 61 K 35/78**

(21) Application number: **80304338.9**

(22) Date of filing: **02.12.80**

(54) **A process for preparing substances having interferon inducing activity.**

(30) Priority: **03.12.79 JP 156627/79**
**31.12.79 JP 172643/79**

(43) Date of publication of application:
**17.06.81 Bulletin 81/24**

(45) Publication of the grant of the patent:
**30.01.85 Bulletin 85/05**

(84) Designated Contracting States:
**CH DE FR GB IT LI**

(56) References cited:
**DE-C-1 000 134**
**FR-A-2 202 683**
**FR-A-2 430 234**
**GB-A- 753 718**
**GB-A-2 036 751**
**GB-A-2 042 555**
**GB-A-2 042 558**
**US-A-2 115 492**

(73) Proprietor: **KITASATO KENKYUSHO**
**9-1, Shirokane 5 chome Minato-ku**
**Tokyo-to (JP)**

(72) Inventor: **Kojima, Yasuhiko**
**244-9, Kosugaya-cho Totsuka-ku**
**Yokohama-shi Kanagawa-ken (JP)**
Inventor: **Tamamura, Sadao**
**1-40-5, Shimouma Setagaya-ku**
**Tokyo-to (JP)**
Inventor: **Konno, Seishi**
**415-1-1203, Kuwakawa-cho Edogawa-ku**
**Tokyo-to (JP)**
Inventor: **Hashimoto, Takashi**
**1-21, Kami-ishihara Chofu-shi**
**Tokyo-to (JP)**

(74) Representative: **Watkins, Arnold Jack et al**
**European Patent Attorney Frank B. Dehn & Co.**
**Imperial House 15-19 Kingsway**
**London WC2B 6UZ (GB)**

Courier Press, Leamington Spa, England.

(56) References cited:

J. Biochem. 212, pp. 607-615 (1975)

J. Exp. Med. 124, pp. 859-872 (1966)

Methods in Carbohydrate Chemistry, Vol. VI, Academic Press, N.Y. (1972), pp. 106-119

"Chinese Herbs; Their Botany, Chemistry and Pharmacodynamics" by John D. KEYS (1976), pp. 208-209, Charles E. Tuttle Comp.

"Shokubutsu Kagaku" (Botanical Chemistry), 4th Ed., pp. 225-226 (1981), Shiiyaku Suppan K.K. Tokyo

"Commentary on the Japanese Pharmacopoeia", 9th Revised Ed., (1976), pp. D 386-388

"Merck Index", 8th Ed., "Alkannin", p. 32

CHEMICAL ABSTRACTS, vol.67, 1967, page 5775, abstract 61622j, Columbus, Ohio, USA, S.C.CHOU et al., "Chemical fractionation of antiviral plants"

## Description

This invention relates to a process for preparing substances having interferon inducing activity. More particularly the process relates to the extraction of such a substance from the tissue of higher plants.

The term interferon inducer used herein denotes a substance capable of acting upon the cell or serum of animals to induce the production of interferon (hereinafter also referred to as IF). Thus an IF inducer is of potential interest in the prevention and treatment of various human and animal diseases caused by viral infection.

It is known that certain mitogenic agents such as phytohemagglutinin [Wheelock, Science, *140*:310 (1965)], poke weed mitogen [Friedman et al, Proc. Soc. Exp. Biol. Med., *125*:90 (1967)] and concanavalin A [Willen et al, Cell. Immunol., *6*:110 (1973)] isolated from the tissue of the kidney bean, poke weed and horse bean, respectively, possess IF inducing activity. However, no successful attempt has been made to obtain an IF inducer from these plants because of their extremely low IF inducing activity. Subsequently, Kojima *et al* disclosed in a Japanese Patent Application laid open to public inspection as Kokai Koho 32107/78 that an IF inducer having high IF inducing activity may be obtained by extraction from the root of *Angelica acutiloba* Kitagawa (*Umbelliferaceae*) known in Japan as *Toki**  of the Family *Seri** (in this specification, terms marked * are those used in Japanese nomenclature). The extraction was effected with hot water to give an extracted solution, to which acetone was added to give a precipitate followed by freeze-drying the precipitate. If desired, the extracted solution may be dehydrated to a suitable quantity by concentration *in vacuo* or by the use of a Diaflo membrane. Kojima and Tamamura disclose in a Japanese Patent Application, laid open to public inspection as Kokai Koho 99313/78, a process for producing an IF inducer, comprising the extraction of the IF inducer from the peeling of the root of a mulberry such as *Morus alba* L. or *M. bombycis* Koidz. The extraction is effected using hot water to give an extracted solution, adding an organic solvent to the solution to give a precipitate, adding a small amount of water to the precipitate subjecting the solution to dialysis to give a residue and freeze-drying the same. If desired, after extraction and/or before dialysis the solution may be dehydrated to give a suitable quantity by concentration *in vacuo* or by using a Diaflo membrane.

As a result of further studies, it has been found that certain substances which we have isolated from the tissues of various higher plants show high IF inducing activity and may be obtained therefrom readily and cheaply. The present invention is based on the discovery that various plants which have been used as traditional remedies in Japan and China are not only effective in regulating general health but are also believed to be effective in curing the common cold. These plants are used empirically on the basis of unique teachings which are completely independent of European pharmacy and accordingly their pharmacodynamic activity has never been clarified. As far as we are aware there has never been any disclosure that these plants contain a substance having interferon inducing activity and no attempt has hitherto been made to extract such a substance except from the above-mentioned *Toki** and mulberries.

The present invention is based on the discovery that a water soluble IF inducer may be extracted from the tissues of higher plants.

Thus according to this invention, there is provided a process for preparing a high molecular weight, water soluble IF inducer characterised in that the said IF inducer is extracted from water from the tissues of a plant selected from the genus *Atractylodes, Lonicera, Plantago, Lithospermum, Ligusticum, Cnidium, Bupleurum, Notopterygium, Heracleum, Aralia, Panax, Polygala, Sophora, Euchresta, Astragalus, Sinomenium, Stephania, Cocculus, Cimicifuga, Rheum, Gastrodia, Asparagus* and *Pinellia* or a variant thereof capable of producing the said IF inducer and recovering the said IF inducer from the extract thereby obtained.

The higher molecular weight interferon inducers produced according to the process of the present invention preferably have a molecular weight of at least 50,000.

The plants of interest are shown hereinafter in Table 1. A variant of such a plant may also be used for the process of this invention when they contain the active substance. The term "variant" used herein denotes a naturally or artificially occurring hybrid or mutant of the parent plant. Preferred plants for use in the process of the present invention are exemplified in Table 2.

## TABLE 1

| Family (subfamily) | Genus |
| --- | --- |
| Compositae | Atractylodes |
| Caprifoliaceae | Lonicera |
| Plantaginaceae | Plantago |
| Borraginaceae | Lithospermum |
| Umbelliferaceae | Ligusticum |
| | Cnidium |
| | Bupleurum |
| | Notopterygium |
| | Heracleum |
| | Angelica |
| Araliaceae | Aralia |
| | Panax |
| Polygalaceae | Polygala |
| Legminosae | Sophora |
| | Euchresta |
| | Astragalus |
| Menispermaceae | Sinomenium |
| | Stephania |
| | Cocculus |
| Ranunclaceae | Cimicifuga |
| Polygonaceae | Rheum |
| Orchidaceae | Gastrodia |
| Liliaceae | Asparagus |
| Araceae | Pinellia |

TABLE 2
Preferred plants and IF induction *in vitro* and *in vivo*

| Example No. | (Family) botanical name | Tissue | In vitro ($\mu$g/ml) | | | In vivo |
|---|---|---|---|---|---|---|
| | | | 10 | 1.0 | 0.1 | |
| | (*Compositae*) *Atractylodes* | | | | | |
| 5 | *ovata* DC | rhizome | >100 | 88 | 30 | 105±35 |
| 6 | *japonica* Koidz. | rhizome | >100 | 92 | 45 | 145±45 |
| 7 | *lancea* DC | rhizome | >100 | 80 | 40 | 120±40 |
| | *chinensis* DC *lancea* DC var. *chinesis* Kitam. *lancea* DC var. *simplicifolia* Kitam. | | | | | |
| | (*Caprifoliaceae*) *Lonicera* | | | | | |
| 8 | *japonica* Thunb. | flower | >100 | >100 | 40 | 95±25 |
| 9 | *confusa* DC | flower | >100 | 90 | 35 | 80±20 |
| 10 | *chinensis* Watson *Lonicera* *maackii* Maxim *similis* Hemsl *tragophylla* Hemsl *Pampaninii* Levl *hypoglauca* Mic. *macranthoides* Hand-Mazz *affinis* Hook et Arn. *flexuosa* Thunb. *brachypoda* DC *chrysantha* Turcz *demissa* Rehd. *morrowii* A. Gray *linderifolia* Max. *strophiophora* Fr. *praeflorens* Batalin | flower | >100 | 95 | 38 | 85±30 |
| | *ramosissima* Fr. et Sav. *caprifolium* L. *xylosteum* L. *sempervirens* Ait. (*Plantaginaceae*) *Plantago* | | | | | |
| 11 | *asiatica* L. | seed | 52 | <10 | <10 | 32±12 |
| 12 | | leaf | >100 | >100 | 50 | 190±65 |
| 13 | *patagonica* | leaf | >100 | >100 | 45 | 185±35 |

TABLE 2 (contd.)

| Example No. | (Family) botanical name | Tissue | In vitro (µg/ml) | | | In vivo |
|---|---|---|---|---|---|---|
| | | | 10 | 1.0 | 0.1 | |
| 14 | *japonica* Fr. | leaf | >100 | >100 | 45 | 175±40 |
| | *lanceolata* L. | | | | | |
| | *mohnikei* Miq. | | | | | |
| | *Camtschatica* Cham. | | | | | |
| | *virginica* L. | | | | | |
| | *Plantago* | | | | | |
| | *major* L. | | | | | |
| | *psyllium* L. | | | | | |
| | *loureiri* Roem et Schult. | | | | | |
| | (*Borraginnaceae*) | | | | | |
| | *Lithospermum* | | | | | |
| 15 | *erythrorhizon* Sieb et Zucc | root | >100 | >100 | 70 | 230±30 |
| 16 | *euchromum* Royle | root | >100 | >100 | 80 | 220±35 |
| | *arvense* L. | | | | | |
| 17 | *officinale* L. | root | >100 | >100 | 75 | 225±40 |
| | *officinale* var. | | | | | |
| | *erythrorhizon* Max. | | | | | |
| | *zollingeri* DC | | | | | |
| | *ruderale* Dougl. | | | | | |
| | (*Umbelliferaceae*) | | | | | |
| | *Ligusticum* | | | | | |
| 18 | *officinale* Kitagawa | rhizome | >100 | >100 | 80 | 180±30 |
| 19 | *chauxiong* Hort | rhizome | >100 | >100 | 50 | 150±25 |
| 20 | *wallichii* Fr. | rhizome | >100 | >100 | 55 | 150±30 |
| | *japonica* Max. | | | | | |
| | *Cnidium* | | | | | |
| | *officinale* Makino | | | | | |
| 21 | *japonica* Miq. | rhizome | >100 | >100 | 55 | 160±30 |
| | *ajanense* Drude | | | | | |
| | *tachiroei* Makino | | | | | |
| | *Bupleurum* | | | | | |
| 22 | *Falcatum* L. | root | >100 | >100 | 85 | 190±32 |
| 23 | *longiradiatum* Turcz | root | >100 | >100 | 64 | 160±40 |
| | *Bupleurum* | | | | | |
| 24 | *scorzonaefolium* | root | >100 | >100 | 55 | 150±40 |
| 25 | *nipponicum* Koso-Poliansky | | | | | |
| | *longeradiatum* var. shikotanensis Ohwi | | | | | |
| | *shikotanensis* Hort | | | | | |
| | *triadiatum* Adams | | | | | |
| | *longeradiatum* var. brevirsdiatum Fr. Scm | | | | | |

**0 030 444**

TABLE 2 (contd.)

| Example No. | (Family) botanical name | Tissue | In vitro (μg/ml) | | | In vivo |
|---|---|---|---|---|---|---|
| | | | 10 | 1.0 | 0.1 | |
| 26 | *Notopterygium* *incisium* Ting | root | >100 | >100 | 92 | 200±20 |
| | *Heracleum* *lanatum* Michx. *hemsleyanum* Michx. *canadium* Fr. *stenopterum* Diels (*Araliaceae*) *Aralia* | | | | | |
| 27 | *cordata* Thunb. | root | >100 | 90 | 35 | 130±28 |
| 28 | *Panax* *ginseng* Meyer | root | >100 | 80 | 10 | 25±5 |
| 29 | *japonicus* Meyer *quinquefolius* L. *pseudo-ginseng* Wall. | root | >100 | 55 | 10 | 22±7 |
| 30 | (*Polygalaceae*) *Polygola* *tenuifolia* Willd. | root | >100 | >100 | 78 | 90±20 |
| 31 | *japonica* Houtt. *tatarinowii* Regel *reinii* Fr. et Sav. (*Legmonosae*) *Sophora* | root | >100 | >100 | 70 | 90±20 |
| 32 | angustifolia Sieb et Zucc | root | >100 | >100 | 85 | 120±40 |
| | *angustifolia* var. *purpurascens* Makino | | | | | |
| 33 | *flavescens* Ait. | root | >100 | >100 | 80 | 110±35 |
| | *flavescens* var. *angustifoila* Kitagawa | | | | | |
| 34 | *subprosarata* Chun et Chen | root | >100 | >100 | 90 | 110±55 |
| 35 | *Euchresta* *japonica* Benth | root | >100 | >100 | 80 | 105±45 |
| 36 | *Astragalus* *membranaceus* Bunge | rhizome | >100 | >100 | 90 | 130±32 |
| 37 | *mongholicus* Bunge *adsurgens* Pall | rhizome | >100 | >100 | 80 | 120±40 |
| 38 | *Astragalus* *sinicus* L. *reflexistipulus* Miq. *yamamotoi Miyake* et al *hoantchy* Fr. | rhizome | >100 | >100 | 80 | 110±40 |
| 39 | (*Menispermaceae*) *Sinomenium* *acutum* Rhed. et Wils. *diversifolium* Diels | root | >100 | >100 | 65 | 270±30 |

7

**0 030 444**

TABLE 2 (contd.)

| Example No. | (Family) botanical name | Tissue | In vitro (µg/ml) | | | In vivo |
|---|---|---|---|---|---|---|
| | | | 10 | 1.0 | 0.1 | |
| 40 | *Stephania*<br>*tetrandra* Moore | root | >100 | >100 | 70 | 250±40 |
| 41 | *Cocclus*<br>*trilobus* DC.<br>*sarmentosus* DC.<br>*laurifolius* DC.<br>*thunbergii* DC. | root | >100 | >100 | 75 | 210±45 |
| 42 | (*Ranunclaceae*)<br>*Cimicifuga*<br>*simplex* Wormkskarl | rhizome | >100 | >100 | >100 | 190±40 |
| 43 | *dahurica* Max. | rhizome | >100 | 80 | 32 | 95±20 |
| 44 | *heracleifolia* Komar. | rhizome | >100 | 75 | 25 | 90±25 |
| 45 | *frigida* Royle<br>*ternata* Miq. | | | | | |
| 46 | *foetida* L.<br>*japonica* Thunb. | rhizome | >100 | 90 | 35 | 100±20 |
| | *Cimicifuga*<br>*racemosa Bart.* | | | | | |
| 47 | (*Polygonaceae*)<br>*Rheum*<br>*palmatum* L. | rhizome | >100 | >100 | 90 | 180±30 |
| 48 | *officinale* Baillon<br>*undulatum* L.<br>*laciniatum* L. | rhizome | >100 | >100 | 50 | 170±30 |
| 49 | *rhaponticum* L.<br>*pontaninii* Los-Losinsk.<br>*enodi* Wall.<br>*franzenbachii* Muent<br>*collinianum* Baillon<br>*speciforme* Royle | rhizome | >100 | >100 | 60 | 160±40 |
| 50 | (*Orchidaceae*)<br>*Gastroida*<br>*elata* Blume<br>*gracilis* Blume<br>*nipponica* Tuyama<br>*acerina* Tanaka<br>*confusa* Hinda et Tuyama | rhizome | >100 | >100 | 95 | 280±55 |
| 51 | (*Liliaceae*)<br>*Asparagus*<br>*lucidus* Lindley | rhizome | >100 | >100 | 45 | 130±35 |
| 52 | *officinalis* L.<br>*medeoloides* Thunb.<br>*racemosus* Willd.<br>*schoberioides* Kunth.<br>*insularis* Hance<br>*falcatus* Benth<br>*oligoclonos* Max. | rhizome | >100 | 95 | 40 | 120±20 |

8

TABLE 2 (contd.)

| Example No. | (Family) botanical name | Tissue | In vitro ($\mu$g/ml) 10 | 1.0 | 0.1 | In vivo |
|---|---|---|---|---|---|---|
| 53 | *cochinchinensis* Merri. (Araceae) *Pinellia* | rhizome | >100 | >100 | 35 | 110±30 |
| 54 | *ternata* Breit. *tripartita* Schott *ternata forma angustata* Makino | rhizome | >100 | >100 | 60 | 140±35 |
| 55 | *tuberifera* Tenore | rhizome | >100 | >100 | 65 | 150±40 |

In this specification, the botanical names are indicated mainly with reference to:—

— "North American Flora", vol. 24 (1924), published by the New York Botanical Garden,
— "Illustrated Flora of The Pacific States", vol. 1 (1940), 2 (1950), 3 (1951) and 4 (1965), published by The Stanford University Press,
— "Coloured Illustration of Herbaceous Plants of Japan", published by Hoikusha, Osaka, Japan (1980) in Japanese version,
— "Chinese Herbs, Their Botany, Chemistry and Pharmacodynamics" by J. D. Keys, published by C. E. Tuttle Company, Rutland, Vermont and Tokyo, Japan (1976),
— "Chinese Medical Herbs", compiled by Li Shih-Chen, translated and researched by F. P. Smith & G. A. Stuart, published by Georgetown Press, San Francisco (1973).

It is preferred to use the dried plant for better extraction efficiency and preservation, although it is possible to use the fresh material if desired.

The extraction may, conveniently, be effected with water at any temperature e.g. from ambient to 120°C, especially when the extraction is effected using the seed. However, a lower temperature may be preferred e.g. from ambient to 75°C in the case of tissues other than the seed. Because the active substances contained in these plants are soluble in water, in particular, under alkaline conditions, it is preferred to adjust the pH of the water before use, for example, to 7—10 e.g. by using a convenient alkali such as sodium hydroxide, potassium hydroxide, ammonium hydroxide or a suitable buffer solution. The extraction may be effected over any convenient period of time sufficient to extract the major portion of the active substance, usually 1—5 days at room temperature, which may be shortened if the extraction temperature is raised. Thus, for example, extraction may be effected for 30 minutes to 6 hours at 45°C to 100°C. In this manner, it is possible to extract the major portion of the active substance contained in the plant tissue (in some cases, more than 90%). However, for example, in the cases of extraction of the plants of the genera *Bupleurum, Notopterygium, Heracleum, Aralia, Polygala, Stellaria, Gastrodia,* it is preferred to extract them at a relatively high temperature e.g. at 80—120°C for 1—2 hours. When the extraction is effected at room temperature with water, a suitable antiseptic agent may, if desired, be added to the extracting water before use. The extraction may be effected intermittently or continuously at any convenient ratio of water to raw material used.

The residue of the raw material is removed from the extracted solution in conventional manner, for example, by filtration, pressing or centrifugation. After this, undesired impurities may be removed from the resultant supernatant in order to allow recovery of the active substance.

Although the physico-chemical characteristics of the IF inducers extracted from various plants have not yet been clarified, they are water soluble and are believed to be high molecular weight substances. According to one embodiment of the invention, the supernatant may be fractionated by ultrafiltration e.g. using a membrane for fractionating substances having a molecular weight of at least 50,000, which may be effected e.g. at 0.1 to 5 kg/cm². The resultant active fractions are collected and combined, and the combined fractions are freeze-dried to yield a crude powder.

It is alternatively possible to recover the active substance from the extracted solution by the use of a hydrophilic organic solvent such as e.g. methanol, ethanol, propanol, butanol or acetone at an appropriate concentration (e.g. 40—90% w/v) so as to form a precipitate containing the active substance, which is then dried to give a crude powder. Instead of the organic solvent, it is also possible to add to the extracted solution an ammonium salt such as e.g. ammonium chloride, ammonium sulfate or cetylmethylammoniumbromide or an inorganic metallic salt such as e.g. zinc chloride or copper chloride at an appropriate concentration (e.g. 20—50% w/v) to form a precipitate which is then desalted in conventional manner, for example, by dialysis, followed by freeze-drying to obtain a crude powder.

The recovery by ultrafiltration is, in general, preferred because on the one hand it is simpler and

9

cheaper in operation and on the other hand it avoids undesired chemical changes in the desired product.

The resultant crude powder may further be purified by a suitable method conventionally used for purification of water-soluble, acidic, high molecular weight substances such as, for example, by column chromatography using a suitable agent for gel filtration or an ion exchanger. In the former case, the elution may be effected with water, although it is possible to use a suitable buffer solution. In the latter case, the elution may be effected with a suitable buffer solution.

Preferred agents for gel filtration are exemplified by Sephadex G-50 to G-200, Sepharose 2B to 6B, Sephacryl S-200 or S-300 (commercial products of Pharmacia Fine Chemicals AB., Sweden), Bio-Gel P-30 to P-300, Bio-Gel A (commercial products of Bio-Rad Laboratories Ltd., U.S.A.), Sagavac (commercial product of Saravac Laboratories Ltd., U.K.), and preferred agents for ion exchange treatment are exemplified by DEAE Sephadex A-25 and A-50 ($Cl^-$ form), QAE Sephadex A-25 and A-50 ($Cl^-$ form), CM Sephadex C-25 and C-50 ($Na^+$ form), SP Sephadex C-25 and C-50 ($Na^+$ form), DEAE Sephacel ($Cl^-$ form), DEAE Sepharose CL-6B ($Cl^-$ form), CM Sephacel CL-6B ($Na^+$ form) (commercial products of Pharmacia Fine Chemicals AB., Sweden) and the like. The words "Sephadex", "Sepharose", "Sephacryl" and "Sephacel" are registered Trade Marks of Pharmacia Fine Chemicals AB, Sweden at least in so far as the United Kingdom is concerned. The word "Bio-Gel" is the registered Trade Mark of Bio-Rad Laboratories Ltd., U.S.A. at least in so far as the United Kingdom is concerned.

It is also possible to use a suitable anion or cation exchange cellulose for the purification.

The substance thus obtained may contain impurities to a greater or lesser degree, although its IF inducing activity may be sufficient for practical purposes. If desired, it is also possible to reduce the amount of impurities further by combining these treatments. For example, a more than 1000 fold increase in the specific activity (on the basis of dry weight) may be obtained by combining the gel filtration with ion exchange treatment.

The following Examples illustrate the invention, in which the dried plant was used as starting material unless otherwise specified.

## Example 1
### *Asparagus lucidus*

The root (1 kg) was washed with water and allowed to stand in water (10 l) at room temperature for 3 days to effect extraction, followed by centrifugation using a basket (3000 r.p.m.) to remove the residue which was washed twice with water (each 5 l). The washing liquid was combined with the supernatant and the combined solutions were made clear by centrifugation (9000 r.p.m.) for 20 minutes. The supernatant was subjected to ultrafiltration using an ultrafilter (Model US-6, commercial product of Bio Engineering K.K., Tokyo) with XM 100A membrane (commercial product of Amicon Corpn., U.S.A.) capable of fractionating substances having a molecular weight of 100,000 at a pressure of 3 kg/cm². The residue was collected and combined, followed by freeze-drying to obtain a whitish powder (2.05 g). This powder (1.5 g) was dissolved in water (5 ml) and transferred to a column packed (4.5×70 cm) with Sephadex G-200 (commercial product of Pharmacia Fine Chemicals AB., Sweden) for gel filtration. The elution was effected with water (600 ml) and the effluent was divided into fractions (each 3 ml). Fraction Nos. 28—53 were collected and combined, and the combined fractions were freeze-dried to obtain a whitish powder (205 mg). For further purification, this powder (100 mg) was dissolved in a 0.01 M tris-HCl buffer solution (pH 7.0; I=0.01; 5 ml) and transferred to a column (2.5×70 cm) packed with DEAE Sephadex A-50 (an ion exchanger) and was eluted with a 0.1 M tris-HCl buffer solution (300 ml; pH 9.0; containing 0.5 M NaCl). The effluent was divided into fractions (each 3 ml) and Fraction Nos. 25—35 were collected and combined, and the combined fractions were desalted by using a visking dialyzing tube or a Diaflo membrane (MW=10,000). After this, the solution was freeze-dried to obtain a whitish amorphous powder (67.5 mg). The IF-inducing activity was determined as follows.

Samples of the final product were used to induce IF in the cells and serum of test animals so as to determine the activity of the IF induced by the method hereinafter described in Experiment 1. The results are shown in Tables 3 and 4, from which it is apparent that the IF-inducing activity is positive.

TABLE 3

| Concentration of sample ($\mu$g/ml) | 10 | 1.0 | 0.1 |
|---|---|---|---|
| Activity *in vitro* | >100 | >100 | >100 |

### TABLE 4

| Activity *in vivo* | Time of collection of blood after administration (hours) | | | | |
|---|---|---|---|---|---|
| | 0 | 1 | 2 | 4 | 6 |
| Rabbit 1 | <10 | 100 | 350 | 60 | 15 |
| Rabbit 2 | <10 | 45 | 200 | 35 | 12 |

Table 4 indicates that by using two rabbits treated by the method hereinafter described in Experiment 1, the IF inducing activity was highest 2 hours after administration. It was also found by the method hereinafter described in Experiment 2, that the test sample comprises an IF inducer.

Example 2
*Sophora angustifolia*

The root (1 kg) was washed with water and put in water (10 l) to effect extraction at room temperature for 2 hours. After this, the extracted solution was treated with 1 N NaOH to adjust the pH to 8.5 and was heated at 60°C for 2 hours to effect further extraction, followed by a similar treatment to that described in Example 1 to give a whitish amorphous powder (72.3 mg). Tables 5 and 6 indicate that the final product shows IF-inducing activity as determined by the method hereinafter described in Experiment 1.

### TABLE 5

| Concentration of sample (μg/ml) | 10 | 1.0 | 0.1 |
|---|---|---|---|
| Activity *in vitro* | >100 | >100 | >98 |

### TABLE 6

| Activity *in vivo* | Time of collection of blood after administration (hours) | | | | |
|---|---|---|---|---|---|
| | 0 | 1 | 2 | 4 | 6 |
| Rabbit 1 | <10 | 75 | 420 | 40 | 12 |
| Rabbit 2 | <10 | 40 | 300 | 18 | 10 |

Example 3
*Bupleurum falcatum*

The root (200 g) was washed with water and then allowed to stand in water (2000 ml). The extraction was effected at room temperature for 2 hours. After this, the solution was treated with 1 N NaOH to adjust the pH to 8.5 and heated at 65°C for 2 hours to effect further extraction. By centrifugation (3000 r.p.m.) using a basket, the filtrate was separated from the residue which was washed twice with water (each 250 ml), and the washing liquid was combined with the filtrate. The combined solution was made clear by centrifugation (9000 r.p.m.). The supernatant was stirred and treated with an aqueous solution of ammonium sulfate (52%; 7500 ml; ×3 by volume). The mixture was stirred at 4°C overnight and was then centrifuged (9000 r.p.m.) for 20 minutes to collect the precipitate formed, which was desalted by subjection to dialysis for 3 days using deionized water and a cellulose tube, followed by freeze-drying to obtain a crude powder (3.25 g). This powder was purified in a similar manner to that described in Example 1 to obtain a whitish amorphous powder (69.2 mg).

Samples of the final product were used to determine the activity of the IF induced in the cells and serum of test animals by the method hereinafter described in Experiment 1. The results shown in Tables 7 and 8 indicate that the IF-inducing activity is positive.

### TABLE 7

| Concentration of sample (μg/ml) | 10 | 1.0 | 0.1 |
|---|---|---|---|
| Activity *in vitro* | >100 | >100 | 98 |

TABLE 8

| Activity in vivo | Time of collection of blood after administration (hours) | | | | |
|---|---|---|---|---|---|
| | 0 | 1 | 2 | 4 | 6 |
| Rabbit 1 | <10 | 40 | 290 | 35 | 10 |
| Rabbit 2 | <10 | 46 | 330 | 13 | |

Example 4
*Pinellia ternata*

The rhizome (200 g) was washed with water and then allowed to stand in water (2000 ml) to effect extraction at room temperature for 2 hours. The solution was then heated at 100°C for 2 hours to effect further extraction. The solution (2500 ml) was treated with acetone (96%; x2 by volume) and stirred at 4°C overnight. After this, the solution was centrifuged (9000 r.p.m.) for 20 minutes to separate off the residue which was dried *in vacuo* to give a crude brown coloured powder (2.85 g). This powder was treated in a similar manner to that described in Example 1 to obtain a whitish powder (58.5 mg).

Examples 5—55

Various plant tissues listed in Table 2 were independently treated in a similar manner to that described in Example 2 except for the use of a membrane capable of fractionating substances having a molecular weight of 50,000 (UK-50, commercially available from Toyo Roshi K.K., Tokyo). Fraction Nos. 25—35 of the effluent of the gel filtration were collected, combined and freeze-dried to give samples which were then used to determine the IF activity induced by the method hereinafter described in Experiment 1. In Table 2, the activity induced in the serum of the test animals *in vivo* was determined 2 hours after administration of the sample (1 mg/rabbit) by using 2 rabbits.

Experiment 1

IF induction with IF inducer and IF assay: [Reference: Y. Kojima's report in Kitasato Arch. Exp. Med., 43:35 (1970)]

(a) IF induction *in vitro*

A rabbit (weight about 1 kg; New Zealand White; SPF) was killed by cardiac puncture and its spleen, bone marrow and lymph node cells were collected and mixed together to prepare a cell suspension ($10^7$ cells/ml) which was divided into fractions (each 1 ml). The samples were independently treated with 10, 1.0 and 0.1 µg/ml of the final product prepared by the method of Example 1. The mixtures were cultured at 25°C for 24 hours, followed by centrifugation to obtain supernatants, each of which was used to determine the IF activity induced.

(b) IF induction *in vivo*

An IF inducer obtained in a similar manner to that described in Example 1 was dissolved in water (500 µg/ml) and injected into the auricular vein of a rabbit (weight about 1 kg; New Zealand White; SPF). 1, 2, 4 and 6 hours after administration, a 2 ml sample of blood was removed on each occasion from the test animal and the serum of each sample was isolated from the blood and used as a sample for determining the activity of the IF induced in the serum.

(c) Determination of IF inducer

In both methods (a) and (b), Vesicular stomatitis virus was used as the challenge virus in order to determine the activity of the IF induced. A monolayer culture of the lined cells of RK-13 of the rabbit was put in a dish and a predetermined amount of the solution obtained by the above-mentioned (a) or (b) was added thereto. The culture was incubated at 37°C overnight with the addition of Vesicular stomatitis virus. The IF activity was determined from the reduction ratio of plaques. The unit of the IF activity is expressed by the reciprocal number of the highest dilution of the sample required for reducing the number of plaques to 50%.

**Claims**

1. A process for preparing a high molecular weight, water soluble IF inducer characterised in that the said IF inducer is extracted with water from the tissue of a plant selected from the genus *Atractylodes, Lonicera, Plantago, Lithospermum, Ligusticum, Cnidium, Bupleurum, Notopterygium, Heracleum, Aralia, Panax, Polygala, Sophora, Euchresta, Astragalus, Sinomenium, Stephania, Cocculus, Cimicifuga, Rheum, Gastrodia, Asparagus* and *Pinellia* or a variant thereof capable of producing the said IF inducer and recovering the said IF inducer from the extract thereby obtained.

2. A process as claimed in claim 1 wherein the plant is: *Asparagus lucidus* Lindley or a variant thereof.

3. A process as claimed in claim 1 wherein the plant is: *Sophora angustifolia* Sieb and Zucc. or a variant thereof.

4. A process as claimed in claim 1 wherein the plant is: *Bupleurum falcatum* Linne or a variant thereof.

5. A process as claimed in claim 1 wherein the plant is: *Pinellia tuberifera* Tenore or a variant thereof.

6. A process as claimed in claim 1 wherein the plant is: *Lonicera japonica* Thunb. or a variant thereof.

7. A process as claimed in claim 1 wherein the plant is: *Lithospermum erythrorhizon* Sieb and Zucc. or a variant thereof.

8. A process as claimed in claim 1 wherein the plant is: *Euchresta japonica* Benth or a variant thereof.

9. A process as claimed in claim 1 wherein the plant is: *Astragalus membranaceus* Bunge or a variant thereof.

10. A process as claimed in claim 1 wherein the plant is: *Sinomenium acutum* Rhed. et Wils. or a variant thereof.

11. A process as claimed in claim 1 wherein the plant is: *Stephania tetrandra* Moore or a variant thereof.

12. A process as claimed in claim 1 wherein the plant is selected from:— *Plantago japonica* Fr. or a variant thereof and *Plantago asiatica* L. or a variant thereof.

13. A process as claimed in any one of the preceding claims wherein the IF inducer is extracted from the tissue of the dried plant.

14. A process as claimed in any one of the preceding claims wherein the extraction is effected by the use of an aqueous solution of an alkali.

15. A process as claimed in any one of the preceding claims, wherein the recovery is effected by formation of a supernatant which is fractionated by ultrafiltration to yield fractions containing the said IF inducer.

16. A process as claimed in claim 15 wherein the ultrafiltration is effected by the use of a membrane capable of retaining substances having a molecular weight of at least 50,000.

17. A process as claimed in any one of claims 1 to 14 wherein the recovery is effected by adding to the extracted solution a hydrophilic organic solvent so as to yield a precipitate containing the said IF inducer.

18. A process as claimed in any one of claims 1 to 14 wherein the recovery is effected by the addition to the extracted solution of one member selected from an ammonium salt and an inorganic metallic salt.

19. A process as claimed in any one of the preceding claims wherein the IF inducer recovered is purified by a method known *per se*, whereby to obtain a water-soluble, acidic, high molecular weight IF inducer in the form of an amorphous whitish powder.


**Patentansprüche**

1. Verfahren zur Herstellung eines wasserlöslichen Interferon-Induktors mit hohem Molekulargewicht, dadurch gekennzeichnet, daß der Interferon-Induktor mit Wasser aus dem Gewebe einer Pflanze extrahiert wird, welche aus der Familie *Atractylodes, Lonicera, Plantago, Lithospermum, Ligusticum, Cnidium, Bupleurum, Notopterygium, Heracleum, Aralia, Panax, Polygala, Sophora, Euchresta, Astragalus, Sinomenium, Stephania, Cocculus, Cimicifuga, Rheum, Gastrodia, Asparagus* und *Pinellia* oder einer Variante derselben ausgewählt wird und fähig ist, den Interferon-Induktor zu produzieren, und daß der Interferon-Induktor aus dem so erhaltenen Extrakt gewonnen wird.

2. Verfahren nach Anspruch 1, dadurch gekennzeichnet, daß die Pflanze *Asparagus lucidus* Lindley oder eine Veriante derselben ist.

3. Verfahren nach Anspruch 1, dadurch gekennzeichnet, daß die Pflanze *Sophora angustifolia* Sieb et Zucc oder eine Variante derselben ist.

4. Verfahren nach Anspruch 1, dadurch gekennzeichnet, daß die Pflanze *Bupleurum falcatum* Linne oder eine Variante derselben ist.

5. Verfahren nach Anspruch 1, dadurch gekennzeichnet, daß die Pflanze *Pinellia tuberifera* Tenore oder eine Variante derselben ist.

6. Verfahren nach Anspruch 1, dadurch gekennzeichnet, daß die Pflanze *Lonicera japonica* Thunb. oder eine Variante derselben ist.

7. Verfahren nach Anspruch 1, dadurch gekennzeichnet, daß die Pflanze *Lithospermum erythrorhizon* Sieb et Zucc. oder eine Variante derselben ist.

8. Verfahren nach Anspruch 1, dadurch gekennzeichnet, daß die Pflanze *Euchresta japonica* Benth oder eine Variante derselben ist.

9. Verfahren nach Anspruch 1, dadurch gekennzeichnet, daß die Pflanze *Astralagun membranaceus* Bunge oder eine Variante derselben ist.

10. Verfahren nach Anspruch 1, dadurch gekennzeichnet, daß die Pflanze *Sinomenium acutum* Rhed. et Wils. oder eine Variante derselben ist.

11. Verfahren nach Anspruch 1, dadurch gekennzeichnet, daß die Pflanze *Stephania tetranda* Moore oder eine Veriante derselben ist.

12. Verfahren nach Anspruch 1, dadurch gekennzeichnet, daß die Pflanze aus *Plantago japonica* Fr. oder einer einer Variante derselben und *Plantago asiatica* L. oder einer Variante derselben ausgewählt wird.

13. Verfahren nach irgendeinem der vorangehenden Ansprüche, dadurch gekennzeichnet, daß der Interferon-Induktor aus dem Gewebe der getrockneten Pflanze extrahiert wird.

14. Verfahren nach irgendeinem der vorangehenden Ansprüche, dadurch gekennzeichnet, daß zum Extrahieren eine wässrige Lösung eines Alkali verwendet wird.

15. Verfahren nach irgendeinem der vorangehenden Ansprüche, dadurch gekennzeichnet, daß zur Gewinnung eine überstehende Flüssigkeit gebildet wird, welche durch Ultrafiltration fraktioniert wird, wobei Fraktionen erhalten werden, welche den Interferon-Induktor enthalten.

16. Verfahren nach Anspruch 15, dadurch gekennzeichnet, daß die Ultrafiltration mit Hilfe einer Membran durchgeführt wird, welche Substanzen mit einem Molekulargewicht von wenigstens 50.000 zurückzuhalten vermag.

17. Verfahren nach einem der Ansprüche 1 bis 14, dadurch gekennzeichnet, daß zur Gewinnung der extrahierten Lösung ein hydrophiles, organisches Lösungsmittel zugesetzt wird, um einen den Interferon-Induktor enthaltenden Niederschlag auszufällen.

18. Verfahren nach einem der Ansprüche 1 bis 14, dadurch gekennzeichnet, daß zur Gewinnung der extrahierten Lösung ein aus einem Ammoniumsalz und einem anorganischen Metallsalz ausgewähltes Glied zugesetzt wird.

19. Verfahren nach irgendeinem der vorangehenden Ansprüche, dadurch gekennzeichnet, daß der gewonnene Interferon-Induktor durch ein an sich bekanntes Verfahren gereinigt wird, um einen wasserlösliche, sauren Interferon-Induktor mit hohem Molekulargewicht in Form eines amorphen weißlichen Pulvers zu erhalten.

**Revendications**

1. Procédé de préparation d'un inducteur d'interféron hydrosoluble à haut poids moléculaire, caractérisé en ce que ledit inducteur d'interféron est extrait à l'eau du tissu d'une plante choisie dans les genres *Atractylodes, Lonicera, Plantago, Lithospermum, Ligusticum, Cnidium, Bupleurum, Notopterygium, Heracleum, Aralia, Panax, Polygala, Sophora, Euchresta, Astragalus, Sinomenium, Stephenia, Cocculus, Cimicifuga, Rheum, Gastrodia, Asparagus* et *Pinellia* ou d'un variant d'une telle plante capable de produire ledit inducteur d'interféron, et à recueillir ledit inducteur d'interféron de l'extrait ainsi obtenu.

2. Procédé selon la revendication 1, dans lequel la plante est *Asparagus lucidus* Lindley ou un variant de cette plante.

3. Procédé selon la revendication 1, dans lequel la plante est *Sophora angustifolia* Sieb et Zucc. ou un variant de cette plante.

4. Procédé selon la revendication 1, dans lequel la plante est *Bupleurum falcatum* Linné ou un variant de cette plante.

5. Procédé selon la revendication 1, dans lequel la plante est *Pinellia tuberifera* Tenore ou un variant de cette plante.

6. Procédé selon la revendication 1, dans lequel la plante est *Lonicera japonica* Thunb. ou un variant de cette plante.

7. Procédé selon la revendication 1, dans lequel la plante est *Lithospermum erythrorhizon* Sieb et Zucc. ou un variant de cette plante.

8. Procédé selon la revendication 1, dans lequel la plante est *Euchresta japonica* Benth ou un variant de cette plante.

9. Procédé selon la revendication 1, dans lequel la plante est *Astragalus membranaceus* Bunge ou un variant de cette plante.

10. Procédé selon la revendication 1, dans lequel la plante est *Sinomenium acutum* Rhed. et Wills, ou un variant de cette plante.

11. Procédé selon la revendication 1, dans lequel la plante est *Stephania tetrandra* Moore ou un variant de cette plante.

12. Procédé selon la revendication 1, dans lequel la plante est choisie entre: *Plantago japonica* Fr. ou un variant de cette plante et *Plantago asiatica* L. ou un variant de cette plante.

13. Procédé selon l'une quelconque des revendications précédentes, dans lequel l'inducteur d'interféron est extrait du tissue de la plante séchée.

14. Procédé selon l'une quelconque des revendications précédentes, dans lequel l'extraction est effectuée en utilisant une solution aqueuse d'un alcali.

15. Procédé selon l'une quelconque des revendications précédentes, dans lequel le produit est recueilli par formation d'une liqueur surnageante qui est fractionnée par ultrafiltration donnant des fractions contenant ledit inducteur d'interféron.

16. Procédé selon la revendication 15, dans lequel l'ultrafiltration est effectuée en utilisant une membrane capable de retenir des substances ayant un poids moléculaire d'au moins 50 000.

17. Procédé selon l'une quelconque des revendications 1 à 14, dans lequel le produit est recueilli par addition à la solution extraite d'un solvant organique hydrophile de manière à former un précipité contenant ledit inducteur d'interféron.

18. Procédé selon l'une quelconque des revendications 1 à 14, dans lequel le produit est recueilli par addition à la solution extraite d'une substance choisie entre un sel d'ammonium et un sel métallique inorganique.

19. Procédé selon l'une quelconque des revendications précédentes, dans lequel l'inducteur d'interféron recueilli est purifié par une opération connue, de manière à obtenir un inducteur d'interféron hydrosoluble acide de haut poids moléculaire sous la forme d'une poudre blanchâtre amorphe.